# EUROPÄISCHE PATENTSCHRIFT

(11) **EP 1 196 094 B1**
(45) Veröffentlichungstag und Bekanntmachung des Hinweises auf die Patenterteilung: **02.01.2003**
(21) Anmeldenummer: 00949232.3
(22) Anmeldetag: 30.06.2000
(51) Int. Cl.: A61B 17/122

(54) **CHIRURGISCHER CLIP**
SURGICAL CLIP
PINCE CHIRURGICALE

(30) Priorität: 28.07.1999 DE 19935418
(43) Veröffentlichungstag der Anmeldung: 17.04.2002
(73) Patentinhaber: Aesculap AG & Co. KG, 78532 Tuttlingen (DE)
(72) Erfinder: HERRMANN, Gebhard, D-78597 Irndorf (DE); NESPER, Markus, D-78532 Tuttlingen (DE); WEISSHAUPT, Dieter, D-78194 Immendingen (DE)
(74) Vertreter: Böhme, Ulrich, Dr. Dipl.-Phys.
(86) Internationale Anmeldenummer: EP0006126
(87) Internationale Veröffentlichungsnummer: WO01008568

(56) Entgegenhaltungen:
- DE-A- 2 952 618
- DE-U- 8 911 948
- DE-U- 29 604 518
- GB-A- 2 161 206
- US-A- 3 805 792
- US-A- 3 827 438

## Beschreibung

Die Erfindung betrifft einen chirurgischen Clip mit zwei relativ zueinander um eine Drehachse verschwenkbaren und in einer Klemmstellung einen im wesentlichen aneinander anliegenden Klemmbereich aufweisenden Klemmarmen, die jeweils ein freies und ein mit einem Lager versehenes Ende aufweisen, wobei in den beiden Lagern eine gemeinsame, die Drehachse definierende Welle gelagert ist, und mit einem den beiden Klemmarmen zugeordneten und diese in der Klemmstellung unter Vorspannung haltenden Spannelement, wobei sich die Lager an der Welle abstützen und das eine der beiden Lager einen von der Welle durchsetzten Lagerring umfaßt.

Bekannt ist beispielsweise ein Clip nach Heifetz, bei dem zwei Cliphälften relativ zueinander verschwenkbar an einem eine Drehachse definierenden Lagerstift gelagert sind. Zusätzlich ist eine den Stift umgebende Torsionsfeder innenliegend mit den beiden Cliphälften verbunden, um diese in der Klemmstellung unter Verspannung zu halten. Derartige Clips werden beispielsweise zum Abklemmen von Hohlorganen verwendet, insbesondere von Blutgefäßen.

Ein solcher Clip besteht demnach aus mindestens vier Bauteilen, nämlich den beiden Cliphälften, der Torsionsfeder und dem Lagerstift. Als nachteilig erweist sich u.a. der Zusammenbau, denn der Lagerstift muß mit den beiden Cliphälften unlösbar verbunden werden, damit er nicht unbeabsichtigterweise herausfallen und verloren gehen kann, wobei gleichzeitig der Clip in seine Bestandteile zerfallen würde.

Ein chirurgischer Clip der eingangs beschriebenen Art ist aus der GB 2 161 206 A bekannt, bei dem die beiden Cliphälften zu einem Lagerstift korrespondierende Bohrungen aufweisen, so daß sich die beiden Cliphälften auf dem als Welle dienenden Lagerstift abstützen.

Ein weiterer chirurgischer Clip ist aus der DE 89 11 948 U1 bekannt. Er umfaßt zwei relativ zueinander schwenkbare Klemmarme, die mittels einer Schraubenfeder zusammengepreßt werden, wobei die freien Enden der Schraubenfeder mit je einer Cliphälfte verbunden sind.

Es ist dementsprechend Aufgabe der vorliegenden Erfindung, einen Clip der eingangs beschriebenen Art derart auszugestalten, daß der konstruktive Aufbau und die Herstellung vereinfacht werden.

Diese Aufgabe wird erfindungsgemäß dadurch gelöst, daß die Welle von dem Spannelement gebildet wird.

Das Spannelement übt demnach bei der vorliegenden Erfindung eine Mehrfachfunktion aus. Es dient der Lagerung der beiden Klemmarme und somit deren Führung und Abstützung, der Momentenerzeugung, um den Clip ohne Einwirkung zusätzlicher äußerer Kräfte in seiner Klemmstellung zu halten und legt außerdem die Dreh- oder Schwenkachse fest. Ferner wird die Zahl der Bauteile von vier auf drei reduziert, denn ein als Welle dienender Lagerstift ist nunmehr überflüssig. Es wird auf diese Weise der Zusammenbau deutlich vereinfacht, denn der typischerweise sehr kleine Lagerstift muß nicht mühsam mit den Klemmarmen verbunden werden. Allein die definierte Anordnung des Spannelement an den beiden Klemmarmen genügt zur Herstellung des Clips. Der eine Klemmarm stützt sich über den Lagerring direkt an der Welle ab, so daß eine Rotation des Lagerrings um die Welle sowie auch eine axiale Verschiebung der Welle in Richtung der Drehachse möglich wird. Es genügt demnach, die Welle relativ zum Klemmarm in Richtung der Drehachse zu sichern, damit die Welle nicht verlorengeht.

Das andere Lager könnte zwar auch einen von der Welle durchsetzten Lagerring umfassen, besonders günstig ist es aber, wenn das andere Lager eine die Welle in Umfangsrichtung nur teilweise umgreifende Lagerschale umfaßt. Dadurch wird der Zusammenbau der beiden Klemmarme besonders einfach, denn die Welle wird durch den Lagerring gesteckt und die Lagerschale an die Welle angelegt oder auch aufgesteckt, je nachdem wie groß ein von der Lagerschale definierter Umfangswinkel ist.

Bei einer bevorzugten Ausführungsform kann vorgesehen sein, daß an dem einen der beiden Klemmarme ein Gegenlager vorgesehen ist, an dem sich der andere der beiden Klemmarme in Richtung der Drehachse abstützt. Beim Öffnen des Clips können Kraftkomponenten bedingt durch das Spannelement in Richtung der Drehachse wirken, so daß die Gefahr bestünde, daß die beiden Klemmarme auseinanderfallen könnten. Durch das Gegenlager wird dies verhindert, denn die in Drehachserichtung wirkenden Kräfte werden durch das Gegenlager aufgenommen.

Dabei ist es besonders vorteilhaft, wenn das Gegenlager durch einen an dem einen der beiden Lager angeordneten Vorsprung gebildet wird, an dem sich das andere Lager in Richtung der Drehachse mindestens einseitig abstützt. Es ist demnach nur erforderlich, das eine Lager so auszubilden, daß es an dem Vorsprung direkt oder indirekt anliegt, so daß das Gegenlager von dem einen Klemmarm über das Spannelement ausgeübte Kräfte in Richtung der Drehachse aufnehmen kann.

Denkbar wäre beispielsweise eine Blattfeder als Spannelement. Günstig ist es aber, wenn das Spannelement durch eine Schraubenfeder gebildet wird. Durch die Dikke eines die Schraubenfeder bildenden Drahtes können die Federkräfte individuell eingestellt werden. Außerdem ist eine Schraubenfeder besonders leicht herzustellen. Ferner kann die in der Regel eine zylindrische Außenform aufweisende Schraubenfeder besonders gut als Welle für die beiden Lager dienen.

Gemäß einer weiteren bevorzugten Ausführungsform kann vorgesehen sein, daß das Spannelement zwei freie Enden aufweist, die sich jeweils an einem Klemmarm abstützen. Auf diese Weise wird gewährleistet, daß das von dem Spannelement erzeugte Drehmoment auf die Klemmarme übertragen wird und diese zusammenhält, so daß die Klemmbereiche der Klemmarme aneinander anliegen.

Denkbar wäre es, das freie Ende des Spannelements unlösbar mit einem der beiden Klemmarme zu verbinden. Besonders vorteilhaft ist es jedoch, wenn mindestens eines der freien Enden des Spannelements abgewinkelt ist und sich an einem der Klemmarme im Bereich des Klemmbereichs an einer dem Klemmbereich abgewandten Seite abstützt. Dadurch entsteht eine lösbare Verbindung zwischen Klemmarm und Spannelement, ohne die Funktion und Wirkung des Spannelements zu beeinträchtigen. Das Abwickeln des Spannelements kann beispielsweise rechtwinklig oder auch U-förmig vorgesehen sein.

Günstig ist es, wenn ein anderes freies Ende des Spannelements abgewinkelt ist und sich an dem anderen Klemmarm im Bereich des Klemmbereichs an einer dem Klemmbereich abgewandten Seite abstützt. Das insbesondere beim Öffnen des Clips von dem Spannelement übertragene Drehmoment kann bei dieser Ausgestaltung beispielsweise im Übergangsbereich zwischen Klemmbereich und Lager der beiden Klemmarme wirken, so daß die Klemmkräfte durch die Wahl des Spannelements und entsprechende Wahl eines derartigen Abstützbereiches individuell einstellbar sind.

Vorteilhafterweise kann jedoch auch vorgesehen sein, daß sich das andere freie Ende des Spannelements an einem an einem der beiden Lager angeordneten Spannelementwiderlager abstützt. Durch Vorsehen des Spannelementwiderlagers wird die Anlagefläche der Spannelementenden an den Klemmarmen im Bereich des Klemmbereichs reduziert, wodurch die Zahl der abstehenden Teile in einem Anlegebereich des Clips reduziert wird.

Günstig ist es dabei, wenn das Spannelementwiderlager an dem Lagerring angeordnet ist. An dem Lagerring ist das Spannelementwiderlager besonders leicht anzuordnen, insbesondere ohne Stabilitätsverluste für den Lagerring hervorzurufen. Die Position des Spannelementwiderlagers kann beliebig über den gesamten Winkelbereich angeordnet werden, was vielfältige Ausgestaltungen ermöglicht.

Besonders vorteilhaft ist es, wenn das Spannelementwiderlager durch eine Ausnehmung gebildet ist. In.die Ausnehmung, beispielsweise eine Bohrung oder eine Einkerbung im bzw. am Lagerring, kann ein freies Ende des Spannelements eingelegt werden, z. B. dadurch, daß es zuvor umgebogen wird.

Grundsätzlich kann vorgesehen sein, daß jeder der beiden Klemmarme wenigstens ein Betätigungselement zum Öffnen des Clips aufweist. Durch Krafteinwirkung auf das Betätigungselement kann ein Drehmoment auf die Klemmarme übertragen werden, so daß das Spannelement einer zusätzlichen Spannkraft unterworfen und der Clip geöffnet wird, d. h. die in der Schlußstellung aneinander anliegenden Klemmbereiche bewegen sich voneinander weg. Je nach Lage der Betätigungselemente an den Klemmarmen kann auf diese Weise eine definierte Kraft zum Öffnen des Clips eingestellt werden.

Bei einer bevorzugten Ausführungsform der Erfindung kann vorgesehen sein, daß die Betätigungselemente an den Lagern angeordnet sind. Bei einer solchen Anordnung in der Nähe der Drehachse muß eine erhöhte Kraft zum Öffnen des Clips aufgewandt werden, so daß ein unbeabsichtigtes Öffnen allein schon durch kleine Kräfte nicht möglich ist.

Vorteilhafterweise kann vorgesehen sein, daß die Betätigungselemente an den Lagern dem Klemmbereich im wesentlichen diametral gegenüberliegend angeordnet sind. Auf diese Weise kann ein Clip mit einer besonders schmalen Ausführungsform gebildet werden. Es ergibt sich beispielsweise eine langgestreckte Bauform, wenn die aneinander anliegenden Klemmbereiche im wesentlichen geradlinig verlaufen. Es ist bei einer solchen Ausgestaltung auch nicht notwendig, zum Öffnen des Clips diesen zu umgreifen. Allein eine Krafteinwirkung auf die Betätigungselemente führt zum Öffnen des Clips.

Günstig ist es, wenn eines der Betätigungselemente in einem Endbereich der Lagerschale angeordnet ist. Insbesondere bei einer den Lagerring seitlich begrenzenden Lagerschale dient das Betätigungselement gleichzeitig als Verbindungselement der beiden Hälften der Lagerschale.

Bei einer bevorzugten Ausführungsform der Erfindung kann auch vorgesehen sein, daß das am anderen Klemmarm angeordnete Betätigungselement außerhalb eines von der Lagerschale begrenzten Bereichs des Lagerrings angeordnet ist. Auf diese Weise wird ein Durchgreifen des Betätigungselements zwischen die Lagerschale vermieden, so daß diese einseitig vollständig geschlossen sein kann, wodurch die Führung des Lagerrings innerhalb der Lagerschale verbessert wird.

Es kann jedoch auch vorgesehen sein, daß das am anderen Klemmarm angeordnete Betätigungselement in einem von der Lagerschale begrenzten Bereich des Lagerrings angeordnet ist. Dadurch kann eine zusätzliche Sicherung der beiden Klemmarme relativ zueinander gebildet werden, insbesondere dann, wenn ein Teil des Betätigungselements in Richtung der Drehachse über die Lagerschale vorsteht.

Die Betätigungselemente könnten beispielsweise durch eine Ausnehmung gebildet werden. Besonders vorteilhaft ist es jedoch, wenn mindestens eines der Betätigungselemente durch einen Betätigungsvorsprung gebildet wird. Ein solcher Vorsprung läßt sich insbesondere mit einem Applikationswerkzeug besonders einfach erfassen.

Gemäß einer weiteren bevorzugten Ausführungsform der Erfindung kann vorgesehen sein, daß die Betätigungselemente Werkzeugaufnahmen umfassen, die eine kugelige Oberfläche aufweisen. Das Applikationswerkzeug kann mit zu den Werkzeugaufnahmen korrespondierenden Werkzeugenden ausgestattet sein, die dann relativ zueinander stets vollständig an den Werkzeugaufnahmen angreifen können, und zwar aufgrund der kugeligen Oberfläche und insbesondere unabhängig von einer Schwenkstellung der Klemmarme.

Vorteilhafterweise kann vorgesehen sein, daß mindestens eine der Werkzeugaufnahmen durch eine im wesentlichen halbkugelförmige Ausnehmung gebildet ist. In eine solche Ausnehmung kann besonders gut ein kugeliges Ende eines Applikationswerkzeugs eingreifen. Außerdem ermöglicht die kugelige Oberfläche, daß der Clip bei bedarf. noch zusätzlich um eine Drehachse verschwenkt werden kann, die durch die Zentren der von den kugeligen Oberflächen definierten Kugelmittelpunkte der kugeligen Enden des Applikationswerkzeugs verläuft. Dies ermöglicht eine individuelle Positionierung des Clips auf besonders einfache Weise.

Günstig ist es, wenn mindestens eine Werkzeugaufnahme durch einen im wesentlichen halbkugelförmigen Vorsprung gebildet ist. Bei dieser Ausführungsform kann die Werkzeugaufnahme besonders einfach von einem Werkzeugende in Form einer hohlen Kugelschale ergriffen werden, jedoch mit allen Vorzügen der kugeligen Ausführungsform, insbesondere einer zusätzlichen Schwenkmöglichkeit, unabhängig von einem Öffnungswinkel der Klemmarme.

Die bislang beschriebenen Ausführungsformen würden es grundsätzlich ermöglichen, einen Öffnungswinkel zwischen den Klemmarmen beliebig groß einzustellen. Besonders vorteilhaft ist es jedoch, wenn ein Begrenzungsanschlag an einem der beiden Klemmarme vorgesehen ist zur Begrenzung des Öffnungswinkels des Clips. Auf diese Weise wird gleichzeitig die Rückstellkraft des Spannelements begrenzt. Eine Anordnungsmöglichkeit wäre beispielsweise, den Begrenzungsanschlag an einem der beiden Lager anzuordnen, so daß der den Klemmbereich aufweisende Abschnitt des Klemmarms mit seiner dem Klemmbereich abgewandten Seite bei Öffnung des Clips an den Begrenzungsanschlag anstoßen kann.

Gemäß einer bevorzugten Ausführungsform der Erfindung kann grundsätzlich vorgesehen sein, daß sich die beiden Klemmarme in einem Übergangsbereich vom Klemmbereich zu den Lagern kreuzen. Auf diese Weise läßt sich festlegen, in welchem Orientierungssinn die beiden Lager relativ zueinander verdreht werden müssen, damit sich der Clip öffnet. Ferner wird der Clip zusätzlich durch dieses Ineinandergreifen der Klemmarme gegen ein Auseinanderfallen gesichert.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht eines ersten Ausführungsbeispiels eines Clips mit gekreuzten Klemmarmen;
- Figur 2:: eine perspektivische Ansicht eines zweiten Ausführungsbeispiels eines Clips mit gekreuzten Klemmarmen;
- Figur 3:: eine perspektivische Ansicht eines dritten Ausführungsbeispiels eines Clips ohne gekreuzte Klemmarme; und
- Figur 4:: eine perspektivische Ansicht eines vierten Ausführungsbeispiels eines Clips ohne gekreuzte Klemmarme.

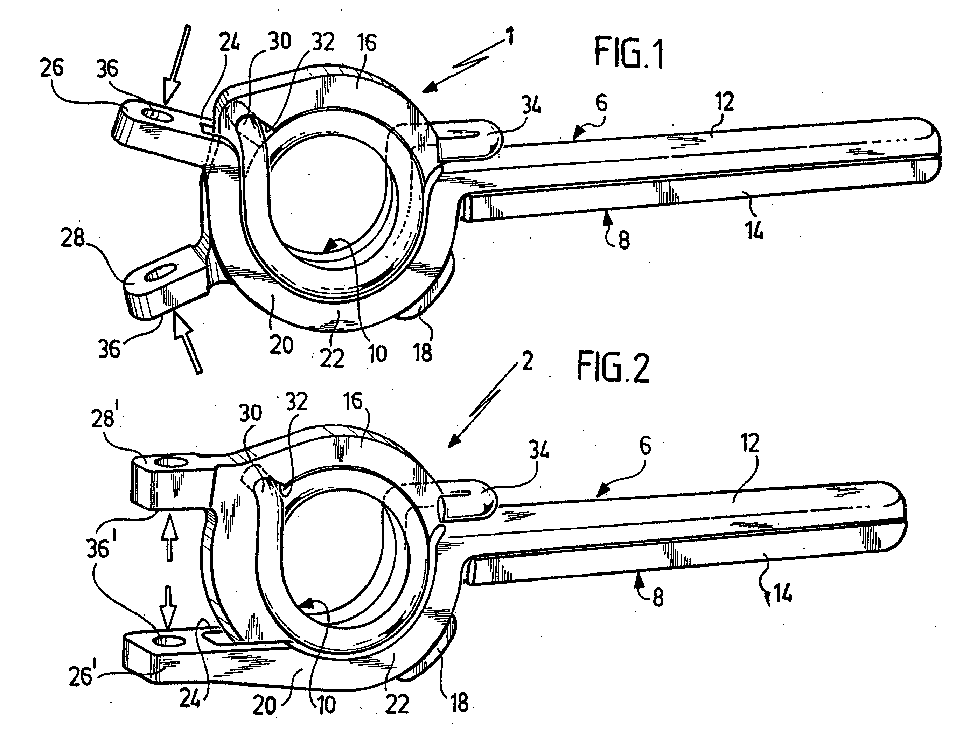

In den Figuren 1 bis 4 sind vier insgesamt mit dem Bezugszeichen 1 bis 4 versehene Clips dargestellt, die jeweils drei Bauteile umfassen, nämlich eine erste Cliphälfte 6, eine zweite Cliphälfte 8 sowie eine Schraubenfeder 10.

Die nachfolgend im einzelnen beschriebenen Ausführungsformen der Clipse 1 bis 4 weisen teilweise einander entsprechende Elemente auf, die aus Gründen der Übersichtlichkeit im folgenden mit denselben Bezugszeichen versehen sind.

Clip 1 weist zwei langgestreckte quaderförmige Klemmarme 12 und 14 auf, die im wesentlichen flächig aneinander anliegen. Die Cliphälfte 8 geht in einem Endbereich des Klemmarmes 14 in einen im wesentlichen kreisförmigen Lagerring 16 über, dessen Symmetrieachse quer zur Längsrichtung des Klemmarmes 14 verläuft. Der Klemmarm 14 verjüngt sich im Bereich des Überganges, so daß der Lagerring 16 etwas dünner als der Klemmarm 14 breit ist.

Unter einem Winkel von etwa 45° bezogen auf den Klemmarm 14 erstreckt sich am Lagerring 16 ein radial abstehendes Gegenlager 18 in Umfangsrichtung über einen Winkelbereich von etwa 25°. Das als Vorsprung ausgebildete Gegenlager 18 durchgreift eine in Richtung der Symmetrieachse des. Lagerrings 16 diesen beidseitig umgreifende Lagerschale 20, die sich an ein Ende des Klemmarms 12 anschließt und deren beide Schalenhälften 22 und 24 beidseitig an dem Lagerring 16 anliegen. Die beiden Schalenhälften 22 und 24 sind an ihren dem Klemmarm 12 abgewandten Enden über einen Betätigungsvorsprung 26 miteinander verbunden. Die Klemmarme 12 und 14 sind bei dem Clip 1 in einer sogenannten gekreuzten Form angeordnet. Dies bedeutet, daß der Klemmarm 14 am Lagerring 16 in einem Bereich angeordnet ist, der zwischen die beiden Schalenhälften 22 und 24 eingreift, weshalb er im Übergangsbereich auch einstufig verjüngt ist.

In ähnlicher Weise kreuzt ein zweiter Betätigungsvorsprung 28 die Lagerschale 20, indem er ebenfalls in einem Bereich am Lagerring 16 angeordnet ist, der sich zwischen den beiden Schalenhälften 22 und 24 befindet. Die Lagerschale 20 erstreckt sich etwa über einen Winkelbereich von 200°, der Betätigungsvorsprung 26 und der Betätigungsvorsprung 28 stehen radial von der Symmetrieachse des Klemmrings 16 weg weisend ab und schließen einen Winkel von etwa 40° ein.

Der Clip 1 wird durch die Schraubenfeder 10 zusammengehalten, deren Außendurchmesser im wesentlichen an den Innendurchmesser des Lagerrings 16 angepaßt ist. Ein freies Ende 30 der Schraubenfeder 10 steht in Schraubenrichtung tangential ab und ist schließlich um 90° abgewinkelt in eine am Lagerring 16 zur Symmetrieachse hin geöffnete Ausnehmung 32 eingelegt. Das andere Ende der Schraubenfeder 10 ist zunächst rechtwinklig parallel verlaufend zum Klemmarm 14 abgewinkelt und umgreift den Lagerring 16 mit einer an dem Klemmarm 12 anliegenden U-förmigen Biegung 34.

Im geschlossenen Zustand des Clips 1, wie er in Figur 1 dargestellt ist, steht die Schraubenfeder 10 bereits unter einer gewissen Vorspannung, so daß die einerseits am Lagerring 16 und andererseits am Klemmarm 12 angreifende Schraubenfeder 10 die beiden Klemmarme 12 und 14 gegeneinander drückt. Die Schraubenfeder 10 dient einerseits als Lagerwelle, auf der der Lagerring 16 und die Lagerschale 20 gelagert sind, wobei sie eine Drehachse definiert, die den Symmetrieachsen des Lagerrings 16 und der Schraubenfeder 10 entspricht.

Der in Figur 1 in seinem geschlossenen Zustand dargestellte doppelt gekreuzte Clip 1 wird geöffnet, indem auf die Betätigungsvorsprünge 26 und 28 eine einander entgegengerichtete Kraft ausgeübt wird, so daß die Betätigungsvorsprünge 26 und 28 zum Öffnen des Clips 1 aufeinander zubewegt werden. Die einander abgewandten Seitenflächen der Betätigungsvorsprünge 26 und 28 sind mit Werkzeugaufnahmen 36 in Form halbkugelförmiger Ausnehmungen versehen. Auf diese Weise kann ein nicht dargestelltes Applikationswerkzeug mit kugelförmigen Werkzeugenden in die Werkzeugaufnahmen 36 eingreifen. Aufgrund der kugeligen Ausgestaltung von Werkzeugaufnahme 36 und Werkzeugende ist in jeder Stellung des Clips 1 eine optimale Kraftübertragung möglich. Zusätzlich läßt sich der Clip 1 bei in die Werkzeugaufnahmen 36 eingreifenden Werkzeugenden um eine Achse verschwenken, die durch die Mittelpunkte der kugeligen Werkzeugenden verläuft. Auf diese Weise läßt sich mit dem Applikationswerkzeug der Clip 1 öffnen und gegebenenfalls in geöffnetem Zustand seitlich verschwenken.

Der in Figur 2 dargestellte Clip 2 unterscheidet sich vom Clip 1 im wesentlichen dadurch, daß nur die Klemmarme 12 und 14 wie bereits oben beschrieben gekreuzt sind. Die Befestigungsvorsprünge 26' und 28' sind nicht gekreuzt, d. h. der Befestigungsvorsprung 28' ist am Lagerring 16 so angeordnet, daß er nicht zwischen den Schalenhälften 22 und 24 der Lagerschale 20 hervorsteht, die sich lediglich über einen Winkelbereich von etwa 150° erstreckt.

Im geschlossenen Zustand des Clips 2 sind die Befestigungsvorsprünge 26' und 28' im wesentlichen parallel zueinander angeordnet und in den einander zugewandten Flächen Werkzeugaufnahmen 36' ebenfalls in Form halbkugeliger Ausnehmungen angeordnet. Zum Öffnen des Clips müssen die kugeligen Werkzeugenden des Applikationswerkzeugs in die Werkzeugaufnahmen 36' geführt und voneinander weggeschwenkt werden. Die Betätigungsrichtung beim Clip 2 ist damit genau umgekehrt zu der beim Clip 1.

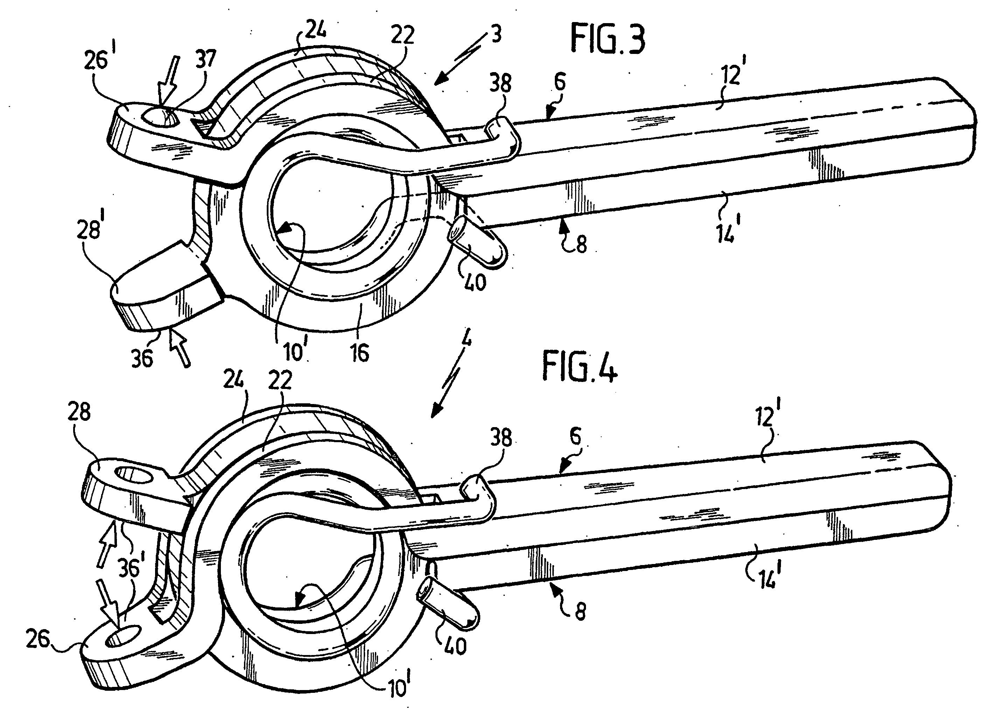

Der in Figur 3 dargestellte Clip 3 weist sowohl ungekreuzte Klemmarme 12' und 14' als auch ungekreuzte Betätigungsvorsprünge 26' und 28' auf. Demnach ist der Klemmarm 12' über die zweiteilige, die Schalenhälften 22 und 24 umfassende und sich über einen Winkelbereich von etwa 150° erstreckende Lagerschale 20 mit dem Betätigungsvorsprung 26' verbunden, der Klemmarm 14' über den Lagerring 16 mit dem Betätigungsvorsprung 28', wobei der Übergang vom Klemmarm 14' zum Lagerring 16 nicht zwischen den Schalenhälften 22 und 24 vorgesehen ist, wie dies bei den Clips 1 und 2 der Fall ist.

Auch beim Clip 3 wird die Lagerwelle durch die Schraubenfeder 10' gebildet, die jedoch gegenüber der bei den Clips 1 und 2 vorgesehenen Schraubenfeder modifizierte Enden 38 und 40 aufweist. Die Schraubenfeder 10' ist einseitig ausgehend von einer letzten Schraubenwindung schräg abstehend in Richtung auf den Klemmarm 12' hin abgebogen, verläuft in einem kurzen Abschnitt im wesentlichen parallel zum Klemmarm 12', ist daran anschließend rechtwinklig abgewinkelt und liegt am Klemmarm 12' auf dessen dem Klemmarm 14' abgewandter Seite auf. Das andere Ende der Schraubenfeder 10' ist in analoger Weise von der anderen Seite um den Lagerring 16 herumgeführt und endet in einem U-förmigen Ende 40, wobei ein quer zu zwei parallel verlaufenden Abschnitten des Endes 40 **verlaufender Steg** am Klemmarm 14' anliegt. Das den Lagerring 16 umgreifende Ende 40 sichert die Schraubenfeder 10' an der Cliphälfte 8 und das Ende 38 die Cliphälfte 6 an der Cliphälfte 8.

Zum Öffnen des Clips 3 müssen die beiden einen Winkel von etwa 45° einschließenden Betätigungsvorsprünge 26' und 28' aufeinander zubewegt werden. Sie weisen an jeweils voneinander weg weisenden Außenflächen halbkugelförmige Vorsprünge 37 auf, die in ein hohlkugelförmiges Werkzeugende eines Applikationswerkzeugs eingreifen können.

Der in Figur 4 dargestellte Clip 4 unterscheidet sich vom Clip 3 dadurch, daß er gekreuzte Betätigungsvorsprünge 26 und 28 aufweist, wie sie auch beim Clip 1 vorgesehen sind, d. h. der Betätigungsvorsprung 28 ist am Lagerring 16 derart abstehend angeordnet, daß er zwischen den Schalenhälften 22 und 24 hervorsteht, die sich über einen Winkelbereich von etwa 200° erstrecken. An einander zugewandten Flächen der Betätigungsvorsprünge 26 und 28 sind wie beim Clip 2 halbkugelig ausgenommene Werkzeugaufnahmen 36' angeordnet. Zum Öffnen des Clips 4 müssen die Betätigungsvorsprünge 26 und 28 voneinander weggeschwenkt werden, beispielsweise dadurch, daß ein Applikationswerkzeug mit kugeligen Werkzeugenden in die Werkzeugaufnahmen 36' eingreift und diese verschwenkt.

Die Werkzeugaufnahmen 36 bzw. 36' können wie beim Clip 3 alternativ auch in Form halbkugeliger Vorsprünge 37 gebildet werden, was eine entsprechend halbkugelig aus-. genommenes Werkzeugende des Applikationswerkzeugs erfordert. In jedem Fall wäre jedoch auch eine Kugelkopfverbindung zwischen Applikationswerkzeug und Werkzeugaufnahme realisiert.

Aufgrund der langgestreckten Bauform der Clips 1 bis 4 ist eine Anwendung durch endoskopische Zugänge möglich. Wichtig zu erwähnen ist, daß die Clips 1 bis 4 zum Öffnen nicht vollständig umgriffen werden müssen, es genügt allein ein Ansetzen des Applikationswerkzeugs an die Werkzeugaufnahmen 36 bzw. 36'.

Je nachdem wie viele Kreuzungen zwischen den Klemmarmen 12 und 14 sowie den Betätigungsvorsprüngen 26 und 28 vorgesehen sind, kann die Bewegungsrichtung zum Öffnen der Clips 1 bis 4 festgelegt werden. Bei einer geraden Anzahl von Kreuzungen öffnet sich der Clip, wenn die Betätigungsvorsprünge 26 und 28 aufeinander zubewegt werden, wie es beispielsweise bei den Clips 1 und 3 der Fall ist, bei einer ungeraden Anzahl von Kreuzungen öffnet sich der Clip, wenn die Betätigungsvorsprünge 26 und 28 voneinander weg bewegt werden.

## Patentansprüche

1. Chirurgischer Clip (1) mit zwei relativ zueinander um eine Drehachse verschwenkbaren und in einer Klemmstellung einen im wesentlichen aneinander anliegenden Klemmbereich aufweisenden Klemmarmen (12, 14), die jeweils ein freies und ein mit einem Lager versehenes Ende aufweisen, wobei in den beiden Lagern eine gemeinsame, die Drehachse definierende Welle gelagert ist, und mit einem den beiden Klemmarmen (12, 14) zugeordneten und diese in der Klemmstellung unter Vorspannung haltenden Spannelement (10), wobei. sich die Lager an der Wolle abstützen und das eine der beiden Lager (16, 20) einen von der Welle (10) durchsetzten Lagerring (16) umfaßt, **dadurch gekennzeichnet**, das die Welle von dem Spannelement (10) gebildet wird.

2. Clip nach Anspruch 1, **dadurch gekennzeichnet, daß** das andere Lager eine die Welle (10) in Umfangsrichtung nur teilweise umgreifende Lagerschale (20) umfaßt.

3. Clip nach einem der voranstehenden Ansprüche, **dadurch gekennzeichnet, daß** an dem einen der beiden Klemmarme (6, 8) ein Gegenlager (18) vorgesehen ist, an dem sich der andere der beiden Klemmarme (6, 8) in Richtung der Drehachse abstützt.

4. Clip nach Anspruch 3, **dadurch gekennzeichnet, daß** das Gegenlager durch einen an dem einen der beiden Lager (16, 20) angeordneten Vorsprung (18) gebildet wird, an dem sich das andere Lager in Richtung der Drehachse mindestens einseitig abstützt.

5. Clip nach einem der voranstehenden Ansprüche, **dadurch gekennzeichnet, daß** das Spannelement durch eine Schraubenfeder (10) gebildet wird.

6. Clip nach einem der voranstehenden Ansprüche, **dadurch gekennzeichnet, daß** das Spannelement (10) zwei freie Enden (30, 34; 38, 40) aufweist, die sich jeweils an einem Klemmarm (6, 8) abstützen.

7. Clip nach Anspruch 6, **dadurch gekennzeichnet, daß** mindestens eines der freien Enden (30, 34; 38, 40) des Spannelements (10) abgewinkelt ist und sich an einem der Klemmarme (6, 8) im Bereich des Klemmbereichs (12, 14) an einer dem Klemmbereich (12, 14) abgewandten Seite abstützt.

8. Clip nach einem der Ansprüche 6 oder 7, **dadurch gekennzeichnet, daß** ein anderes freies Ende (30, 34; 38, 40) des Spannelements (10) abgewinkelt ist und sich an dem anderen Klemmarm (6, 8) im Bereich des Klemmbereichs (12, 14) an einer dem Klemmbereich (12, 14) abgewandten Seite abstützt..

9. Clip nach einem der Ansprüche **6** oder **7, dadurch gekennzeichnet, daß** sich das andere freie Ende (30) des Spannelements (10) an einem an einem der beiden Lager (16, 20) angeordneten Spannelementwiderlager (32) abstützt.

10. Clip nach Anspruch **9, dadurch gekennzeichnet, daß** das Spannelementwiderlager (32) an dem Lagerring (16) angeordnet ist.

11. Clip nach einem der Ansprüche **9** oder **10**, **dadurch gekennzeichnet, daß** das Spannelementwiderlager durch eine Ausnehmung (32) gebildet ist.

12. Clip nach einem der voranstehenden Ansprüche, **dadurch gekennzeichnet, daß** jeder der beiden Klemmarme (6, 8) wenigstens ein Betätigungselement (26, 28) zum öffnen des Clips (1; 2; 3; 4) aufweist.

13. Clip nach Anspruch **12, dadurch gekennzeichnet, daß** die Betätigungselemente (26, 28) an den Lagern (16, 20) angeordnet sind.

14. Clip nach Anspruch **13, dadurch gekennzeichnet, daß** die Betätigungselemente (26, 28) an den Lagern (16, 20) dem Klemmbereich (12, 14) im wesentlichen diametral gegenüberliegend angeordnet sind.

15. Clip nach Anspruch **2** und einem der Ansprüche **13** oder **14, dadurch gekennzeichnet, daß** eines der Betätigungselemente (26, 28) in einem Endbereich der Lagerschale (20) angeordnet ist.

16. Clip nach den Ansprüchen **2** und **14**, **dadurch gekennzeichnet, daß** das am anderen Klemmarm (8) angeordnete Betätigungselement (28) außerhalb eines von der Lagerschale (20) begrenzten Bereichs des Lagerrings (16) angeordnet ist.

17. Clip nach den Ansprüchen **2** und **15, dadurch gekennzeichnet, daß** das am anderen Klemmarm (8) angeordnete Betätigungselement (28) an einem von der Lagerschale (20) begrenzten Bereich des Lagerrings (16) angeordnet ist.

18. Clip nach einem der Ansprüche 12 bis 17, **dadurch gekennzeichnet, daß** mindestens eines der Betätigungselemente durch einen Betätigungsvorsprung (26, 28) gebildet wird.

19. Clip nach einem der Ansprüche 12 bis **18, dadurch gekennzeichnet, daß** die Betätigungselemente (26, 28) Werkzeugaufnahmen (36, 37) umfassen, die eine kugelige Oberfläche aufweisen.

20. Clip nach Anspruch 19, **dadurch gekennzeichnet, daß** mindestens eine der Werkzeugaufnahmen durch eine im wesentlichen halbkugelförmig Ausnehmung (36) gebildet ist.

21. Clip nach einem der Ansprüche **19** oder **20, dadurch gekennzeichnet, daß** mindestens eine der Werkzeugaufnahmen durch einen im wesentlichen halbkugelförmigen Vorsprung (37) gebildet ist.

22. Clip nach einem der voranstehenden Ansprüche, **dadurch gekennzeichnet, daß** sich die beiden Klemmarme (6, 8) in einem Übergangsbereich vom Klemmbereich (12, 14) zu den Lagern (16, 20) kreuzen.

## Claims

1. A surgical clip (1) with two clamping arms (12, 14) swivellable relative to one another about an axis of rotation and having, in a clamping position, a clamping region [in which they are] substantially in contact with one another, each of which arms has a free end and an end provided with a bearing, a common shaft defining the axis of rotation being mounted in the two bearings, and with a tensioning member (10) associated with the two clamping arms (12, 14) and holding them under pre-stress in the clamping position, the bearings being supported on the shaft and one of the two bearings (16, 20) comprising a roller race (16) penetrated by the shaft (10), **characterised in that** the shaft is formed by the tensioning member (10).

2. A clip according to Claim 1, **characterised in that** the other bearing comprises a bearing shell (20) only partially encompassing the shaft (10) in the circumferential direction.

3. A clip according to one of the preceding claims, **characterised in that** a thrust bearing (18) is provided on one of the two clamping arms (6, 8) against which thrust bearing the other of the two clamping arms (6, 8) is supported in the direction of the axis of rotation.

4. A clip according to Claim 3, **characterised in that** the thrust bearing is formed by a projecting part (18) provided on one of the two bearings (16, 20), on which projecting part the other bearing is supported at least on one side in the direction of the axis of rotation.

5. A clip according to one of the preceding claims, **characterised in that** the tensioning member is formed by a helical spring (10).

6. A clip according to one of the preceding claims, **characterised in that** the tensioning member (10) has two free ends (30, 34; 38, 40), each supported on a clamping arm (6, 8).

7. A clip according to Claim 6, **characterised in that** at least one of the free ends (30, 34; 38, 40) of the tensioning member (10) is bent and is supported on one of the clamping arms (6, 8) in the region of the clamping region (12, 14) on a side facing away from the clamping region (12, 14).

8. A clip according to one of Claims 6 or 7, **characterised in that** another free end (30, 34; 38, 40) of the tensioning member (10) is bent and is supported on the other clamping arm (6, 8) in the region of the clamping region (12, 14) on a side facing away from the clamping region (12, 14).

9. A clip according to one of Claims 6 or 7, **characterised in that** the other free end (30) of the tensioning member (10) is supported on a tensioning-member abutment (32) positioned on one of the two bearings (16, 20).

10. A clip according to Claim 9, **characterised in that** the tensioning-member abutment (32) is positioned on the roller race (16).

11. A clip according to one of Claims 9 or 10, **characterised in that** the tensioning-member abutment is formed by a recess (32).

12. A clip according to one of the preceding claims, **characterised in that** each of the two clamping arms (6, 8) has at last one actuating member (26, 28) for opening the clip (1; 2; 3; 4).

13. A clip according to Claim 12, **characterised in that** the actuating members (26, 28) are arranged on the bearings (16, 20).

14. A clip according to Claim 13, **characterised in that** the actuating members (26, 28) on the bearings (16, 20) are arranged substantially diametrically opposite the clamping region (12, 14).

15. A clip according to Claim 2 and one of Claims 13 or 14, **characterised in that** one of the actuating members (26, 28) is positioned on an end region of the bearing shell (20).

16. A clip according to Claims 2 and 14, **characterised in that** the actuating member (28) positioned on the other clamping arm (8) is positioned outside a region of the roller race (16) delimited by the bearing shell (20).

17. A clip according to Claims 2 and 15, **characterised in that** the actuating member (28) positioned on the other clamping arm (8) is positioned on a region of the roller race (16) delimited by the bearing shell (20).

18. A clip according to one of Claims 12 to 17, **characterised in that** at least one of the actuating members is formed by an actuating projection (26, 28).

19. A clip according to one of Claims 12 to 18, **characterised in that** the actuating members (26, 28) comprise tool holding fixtures (36, 37) having a spherical surface.

20. A clip according to Claim 19, **characterised in that** at least one of the tool holding fixtures is formed by a substantially hemispherical recess (36).

21. A clip according to one of Claims 19 or 20, **characterised in that** at least one of the tool holding fixtures is formed by a substantially hemispherical projecting part (37).

22. A clip according to one of the preceding claims, **characterised in that** the two clamping arms (6, 8) cross over one another in a transitional region from the clamping region (12, 14) to the bearings (16, 20).

## Revendications

1. Pince chirurgicale (1), avec deux bras de serrage (12, 14) qui peuvent pivoter l'un par rapport à l'autre autour d'un axe de rotation, présentent dans une position de serrage une zone de serrage essentiellement en application mutuelle, et présentent chacun une extrémité libre et une extrémité pourvue d'un palier, un arbre commun définissant l'axe de rotation étant monté dans les deux paliers, et avec un élément tendeur (10) associé aux deux bras de serrage (12, 14) et les maintenant sous précontrainte dans la position de serrage, les paliers s'appuyant contre l'arbre et l'un des deux paliers (16, 20) comprenant une bague de palier (16) traversée par l'arbre (10), **caractérisée en ce que** l'arbre est formé par l'élément tendeur (10).

2. Pince selon la revendication 1, **caractérisée en ce que** l'autre palier comprend un coussinet (20) qui n'entoure l'arbre (10) que partiellement en direction circonférentielle.

3. Pince selon l'une des revendications précédentes, **caractérisée en ce qu'**un contre-appui (18) est prévu sur l'un des deux bras de serrage (6, 8), contre-appui contre lequel l'autre des deux bras de serrage (6, 8) s'appuie dans la direction de l'axe de rotation.

4. Pince selon la revendication 3, **caractérisée en ce que** le contre-appui est formé par une saillie (18) disposée sur l'un des deux paliers (16, 20), saillie contre laquelle l'autre palier s'appuie au moins d'un côté dans la direction de l'axe de rotation.

5. Pince selon l'une des revendications précédentes, **caractérisée en ce que** l'élément tendeur est formé par un ressort à boudin (10).

6. Pince selon l'une des revendications précédentes, **caractérisée en ce que** l'élément tendeur (10) présente deux extrémités libres (30, 34 ; 38, 40), qui s'appuient respectivement contre un bras de serrage (6, 8).

7. Pince selon la revendication 6, **caractérisée en ce qu'**au moins une des extrémités libres (30, 34 ; 38, 40) de l'élément tendeur (10) est coudée et s'appuie contre un des bras de serrage (6, 8) dans la région de la zone de serrage (12, 14), sur un côté opposé à la zone de serrage (12, 14).

8. Pince selon la revendication 6 ou 7, **caractérisée en ce qu'**une autre extrémité libre (30, 34 ; 38, 40) de l'élément tendeur (10) est coudée et s'appuie contre l'autre bras de serrage (6, 8) dans la région de la zone de serrage (12, 14), sur un côté opposé à la zone de serrage (12, 14).

9. Pince selon la revendication 6 ou 7, **caractérisée en ce que** l'autre extrémité libre (30) de l'élément tendeur (10) s'appuie contre un contre-appui (32) d'élément tendeur disposé sur un des deux paliers (16, 20).

10. Pince selon la revendication 9, **caractérisée en ce que** le contre-appui (32) d'élément tendeur est disposé sur la bague de palier (16).

11. Pince selon la revendication 9 ou 10, **caractérisée en ce que** le contre-appui d'élément tendeur est formé par un évidement (32).

12. Pince selon l'une des revendications précédentes, **caractérisée en ce que** chacun des deux bras de serrage (6, 8) présente au moins un élément d'actionnement (26, 28) pour ouvrir la pince (1 ; ; 2 ; 3 ; 4).

13. Pince selon la revendication 12, **caractérisée en ce que** les éléments d'actionnement (26, 28) sont disposés sur les paliers (16, 20).

14. Pince selon la revendication 13, **caractérisée en ce que** les éléments d'actionnement (26, 28) sont disposés sur les paliers (16, 20) en étant sensiblement diamétralement opposés à la zone de serrage (12, 14).

15. Pince selon la revendication 2 et la revendication 13 ou 14, **caractérisée en ce qu'**un des éléments d'actionnement (26, 28) est disposé dans une région terminale du coussinet (20).

16. Pince selon les revendications 2 et 14, **caractérisée en ce que** l'élément d'actionnement (28) disposé sur l'autre bras de serrage (8) est disposé en dehors d'une région de la bague de palier (16) qui est délimitée par le coussinet (20).

17. Pince selon les revendications 2 et 15, **caractérisée en ce que** l'élément d'actionnement (28) disposé sur l'autre bras de serrage (8) est disposé sur une région de la bague de palier (16) qui est délimitée par le coussinet (20).

18. Pince selon l'une des revendications 12 à 17, **caractérisée en ce qu'**au moins un des éléments d'actionnement est formé par une saillie d'actionnement (26, 28).

19. Pince selon l'une des revendications 12 à 18, **caractérisée en ce que** les éléments d'actionnement (26, 28) comprennent des éléments récepteurs d'outil (36, 37) qui présentent une surface sphérique.

20. Pince selon la revendication 19, **caractérisée en ce qu'**au moins un des éléments récepteurs d'outil est formé par un évidement (36) essentiellement hémisphérique.

21. Pince selon la revendication 19 ou 20, **caractérisée en ce qu'**au moins un des éléments récepteurs d'outil est formé par une saillie (37) essentiellement hémisphérique.

22. Pince selon l'une des revendications précédentes, **caractérisée en ce que** les deux bras de serrage (6, 8) se croisent dans une région de transition entre la zone de serrage (12, 14) et les paliers (16, 20).
